(19) Europäisches Patentamt
European Patent Office
Office européen des brevets

(11) Veröffentlichungsnummer: **0 182 330 B1**

# (12) EUROPÄISCHE PATENTSCHRIFT

(45) Veröffentlichungstag der Patentschrift: **15.05.91**

(51) Int. Cl.5: **A61K 7/13**, C07C 211/48, C07C 215/48, C07C 215/74

(21) Anmeldenummer: **85114609.2**

(22) Anmeldetag: **16.11.85**

Die Akte enthält technische Angaben, die nach dem Eingang der Anmeldung eingereicht wurden und die nicht in dieser Patentschrift enthalten sind.

## (54) Verwendung von 2-Nitroanilinderivaten in Haarfärbemitteln und neue 2-Nitroanilinderivate.

(30) Priorität: **23.11.84 DE 3442757**

(43) Veröffentlichungstag der Anmeldung:
**28.05.86 Patentblatt 86/22**

(45) Bekanntmachung des Hinweises auf die Patenterteilung:
**15.05.91 Patentblatt 91/20**

(84) Benannte Vertragsstaaten:
**AT BE CH DE FR GB IT LI LU NL SE**

(56) Entgegenhaltungen:
**DE-A- 2 645 125**
**DE-A- 2 717 766**
**GB-A- 2 082 207**
**DE-A- 2 714 437**
**DE-A- 3 237 219**
**GB-A- 2 150 148**

(73) Patentinhaber: **Wella Aktiengesellschaft**
**Berliner Allee 65**
**W-6100 Darmstadt(DE)**

(72) Erfinder: **Konrad, Eugen**
**Mecklenburger Strasse 101**
**W-6100 Darmstadt(DE)**
Erfinder: **Clausen, Thomas, Dr.**
**Ernst Pasqué Strasse 35A**
**W-6146 Alsbach(DE)**
Erfinder: **Braun, Hans-Jürgen, Dr.**
**Impasse de la Colline 3**
**CH-1723 Marly(CH)**
Erfinder: **Mager, Herbert, Dr.**
**Beaumont 5**
**CH-1700 Fribourg(CH)**

**EP 0 182 330 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist die Verwendung von Nitrofarbstoffen zum Färben von Haaren, wobei als Nitrofarbstoffe bestimmte 2-Nitroanilinderivate verwendet werden, sowie neue 2-Nitroanilinderivate.

Nitrofarbstoffe finden heute in Haarfärbemitteln eine breite Anwendung. Sie werden in Oxidationshaarfärbemitteln als Zusätze für die Erzeugung von natürlichen oder modischen Farbnuancen verwendet. Durch Kombination von mehreren verschieden gefärbten Nitrofarbstoffen ist es jedoch auch möglich, Haarfärbemittel herzustellen, die Haare in natürlichen bis modischen Nuancen ohne die Anwendung von Oxidationsmitteln zu färben vermögen.

So können zum Beispiel durch die Kombination eines orangefärbenden mit einem blaufärbenden Nitrofarbstoff natürlich wirkende braune Färbungen erzeugt werden. Daneben ist es jedoch auch möglich, mit einem gelbfärbenden und einem violettfärbenden Nitrofarbstoff ein ähnliches Ergebnis zu erhalten. Es werden daher gelbe Nitrofarbstoffe benötigt, die das Haar entweder in einem intensiven reinen Zitronengelb einzufärben vermögen, welches möglichst frei von Rotanteilen sein muß, oder solche, die orange färben und in Kombination mit rein blauen Farbstoffen eingesetzt werden können.

Daneben sind noch viele weitere Anforderungen gestellt. Die Nitrofarbstoffe müssen in toxikologischer und dermatologischer Hinsicht unbedenklich sein. Ihre Verwendung in Oxidationshaarfärbemitteln setzt voraus, daß sie in Anwesenheit von Wasserstoffperoxid in alkalischer Lösung stabil sind. Außerdem wird für die erzeugten Haarfärbungen eine gute Licht-, Säure- und Reibechtheit gefordert. Schließlich sollten die Nitroverbindungen durch möglichst einfache Verfahren darstellbar sein.

Die bisher in der Literatur zur Gelbfärbung des Haares beschriebenen substituierten Amino-nitrophenole erfüllen die vorstehend genannten Voraussetzungen nur unzureichend. Das im International Journal of Cosmetic Science 1982, Seiten 25 - 35 genannte 4-Nitro-3-(2'-hydroxyethylamino)-phenol ergibt zwar eine zitronengelbe Färbung, diese ist aber sehr farbschwach. Zwei weitere Isomere, nämlich das 4-Nitro- und das 5-Nitro-2-(2'-hydroxyethylamino)-phenol, sind keine gelbfärbenden, sondern orangefärbende Nitrofarbstoffe, die aber aufgrund der aromatischen Hydroxygruppen pH-empfindlich sind und unerwünschte Farbänderungen bei Säure- und Alkalieinwirkung zeigen.

Weitere bekannte gelbe Nitrofarbstoffe sind die in der DE-AS 1 619 395 genannten o-, m- und p-Nitroanilinderivate. Diese Verbindungen erfüllen zwar weitgehend die Anforderungen in anwendungstechnischer Hinsicht, befriedigen aber nicht im Hinblick auf die physiologischen Eigenschaften.

Das in der DE-A-3 237 219 genannte 2-N-$\beta$-Hydroxyethylamino-5-methoxy-nitrobenzol färbt die Haare rot und ist mutagen, während das in der DE-A 2 717 766 beschriebene 3-Nitro-4-N-$\beta$-Hydroxyethylaminophenol die Haare rot bis rosa färbt.

Aus der GB-A-2 082 207 sind die Verbindungen 4-N-$\beta$-Hydroxyethylamino-3-methoxy-nitrobenzol und 2-N-$\beta$-Hydroxyethylamino-4-methoxy-nitrobenzol, welche gelb färben und nicht oder wenig mutagen sein sollen, bekannt.

Es wurde nun überraschenderweise gefunden, daß sich die genannten Nachteile beseitigen lassen durch die Verwendung von 2-Nitroanilinderivaten der allgemeinen Formel I

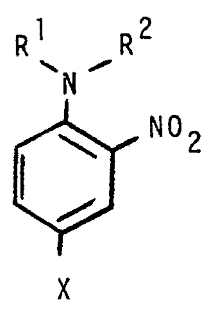

(I),

wobei $R^1$ und $R^2$ die Bedeutung H, $C_1$-$C_4$-Alkyl, $C_2$-$C_4$-Monohydroxyalkyl oder $C_3$-$C_4$-Dihydroxyalkyl haben - unter der Voraussetzung, daß $R^1$ und $R^2$ nicht gleichzeitig $C_1$-$C_4$-Alkylreste darstellen- und X einen der Reste Alkyl, Monohydroxyalkyl, Perfluoralkyl oder Halogen bedeutet, als Farbstoff in Haarfärbemitteln.

Durch Variation des Restes X stehen Verbindungen der Formel I zur Verfügung, die alle benötigten Farbtöne, von einem blaustichigen Zitronengelb über ein reines Gelb bis zu Orange, liefern.

Von den unter die Formel I fallenden Verbindungen ist aus färberischen und physiologischen Gründen bevorzugt die Verwendung von 2-Nitroanilinderivaten der allgemeinen Formel II

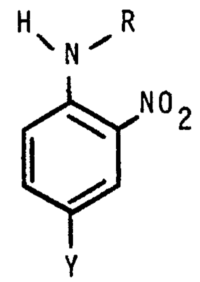

(II),

wobei R die Bedeutung 2-Hydroxyethyl oder 2,3-Dihydroxypropyl hat und Y einen der Reste $CH_3$, $CF_3$, $CH_2OH$, Cl oder Br darstellt, als Farbstoff in Haarfärbemitteln.

Beispiele für erfindungsgemäß geeignete 2-Nitroanilinderivate gemäß der allgemeinen Formel I sind 4-Amino-3-nitrotoluol, 4-Amino-3-nitro-benzylalkohol, 4-Amino-3-nitro-1-trifluormethyl-benzol, 4-Amino-3-nitro-chlorbenzol und 4-Amino-3-nitro-brombenzol, 4-[Bis-(2'-hydroxyethyl)-amino]-3-nitro-toluol, 4-[N-Ethyl,N-(2'-hydroxyethyl)-amino]-3-nitro-toluol, 4-[Bis-(2'-hydroxyethyl)-amino]-3-nitro-chlorbenzol, 4-[N-Ethyl,N-(2'-hydroxyethyl)-amino] -3-nitro-chlorbenzol.

Bevorzugte Vertindungen gemäß der allgemeinen Formel II sind zum Beispiel 4-(2'-Hydroxyethyl)-amino-3-nitro-toluol, 4-(2'-Hydroxyethyl)-amino-3-nitro-benzylalkohol, 4-(2'-Hydroxyethyl)-amino-3-nitro-1-trifluormethyl-benzol, 4-(2',3'-Dihydroxypropyl)-amino-3-nitro-1-trifluormethylbenzol, 4-(2'-Hydroxyethyl)-amino-3-nitro-chlorbenzol, 4-(2'-Hydroxyethyl)-amino-3-nitro-brombenzol und 4-(2',3'-Dihydroxypropyl)-amino-3-nitro-brombenzol.

Überraschend sind die sehr guten toxikologischen und physiologischen Eigenschaften der Verbindungen nach der allgemeinen Formel I. So sind die Farbstoffe mit einer Hydroxyethylaminogruppe und X = Methyl, Trifluormethyl, Chlor und Brom im Amestest nicht mutagen und werden daher und aus Gründen der Löslichkeit und der Farbtiefe auch gegenüber den nicht hydroxyalkylierten Nitroanilinderivaten besonders bevorzugt.

Die erfindungsgemäßen neuen Verbindungen der allgemeinen Formel I stellen für die Färbung von menschlichen Haaren hervorragend geeignete gelbfärbende Nitrofarbstoffe dar. Sie sind gut in Wasser löslich und weisen eine ausgezeichnete Lagerstabilität auf.

Gegenstand der vorliegenden Anmeldung sind daher ebenfalls Mittel zur Färbung von Haaren mit einem Farbstoffgehalt und üblichen kosmetischen Zusätzen, dadurch gekennzeichnet, daß sie ein 2-Nitroanilinderivat der allgemeinen Formel I enthalten. Von diesen Mitteln sind wiederum solche bevorzugt, welche ein 2-Nitroanilinderivat der Formel II enthalten.

Die erfindungsgemäßen Mittel zur Färbung von Haaren betreffen sowohl solche, die ohne Zugabe eines Oxidationsmittels angewendet werden, als auch solche, bei denen die Zugaben eines Oxidationsmittels erforderlich ist.

Bei den ersteren Haarfärbemitteln ohne Oxidationsmittel-Zugabe handelt es sich um diejenigen, die neben den Farbstoffen der angegebenen Formel noch andere direkt auf das Haar aufziehende Farbstoffe enthalten können. Von diesen für die Haarfärbung bekannten Farbstoffen seien beispielsweise die folgenden erwähnt: Aromatische Nitrofarbstoffe, wie zum Beispiel 2-Amino-4-nitro-phenol, Pikraminsäure, 1-[(2'-Hydroxyethyl)-amino]-2-amino-4-nitro-benzol, 2-Nitro-4-[(2'-hydroxyethyl)-amino]-anilin, 4-Bis-[(2'-hydroxyethyl)-amino]-1-methylamino-2-nitro-benzol, 2,5-Bis-[(2'-hydroxyethyl)-amino]-nitrobenzol,2-(2'-Hydroxyethyl)-amino-4,6-dinitrophenol und 1-Amino-4-(2',3'-dihydroxypropyl)-amino-2-nitro-5-chlor-benzol ; Triphenylmethanfarbstoffe, wie zum Beispiel Basic Violet 1 (C.I. 42 535); Azofarbstoffe, wie zum Beispiel Acid Brown 4 (C.I. 14 805); Anthrachinonfarbstoffe, wie zum Beispiel Disperse Blue 23 (C.I. 61 545), Disperse Violet 4 (C.I. 61 105), 1,4,5,8-Tetraaminoanthrachinon und 1,4-Diaminoanthrachinon, wobei die Farbstoffe dieser Klassen je nach Art ihrer Substituenten sauren, nicht-ionogenen oder basischen Charakter haben können Weitere geeignete direkt auf das Haar aufziehende Farbstoffe sind beispielsweise in dem Buch von J.C. Johnson, "Hair Dyes" Noyes Data Corp. Park-Ridge (USA) (1973), Seiten 3 - 91 und 113 - 139 (ISBN: 0-8155-0477-2) beschrieben.

Die Zubereitungsform der hier beschriebenen Haarfärbemittel auf der Basis von direkt auf das Haar aufziehenden Farbstoffen kann beispielsweise eine Lösung, insbesondere eine wäßrige oder wäßrig-alkoholische Lösung, sein. Bevorzugte Zubereitungsformen sind weiterhin eine Creme, ein Gel oder eine Emulsion, wobei sie auch im Gemisch mit einem Treibgas oder mittels einer Pumpe versprüht werden können.

Die Farbstoffe der angegebenen Formeln I und II sollen in diesen Färbemitteln ohne Oxidationsmittel-Zugabe in einer Konzentration von etwa 0,01 bis 2,0 Gew.%, vorzugsweise von 0,01 bis 1,0 Gew.%, enthalten sein. Der Gesamtgehalt an den direkt ziehenden Farbstoffen liegt in den Grenzen von etwa 0,01 bis 3,0 Gew.%.

Der pH-Wert dieser Färbemittel liegt im Bereich von 3 bis 10,5, insbesondere bei pH 7,5 bis 9,5, wobei die Einstellung des gewünschten alkalischen pH-Wertes nauptsächlich mit Ammoniak erfolgt, jedoch auch mit organischen Aminen wie beispielsweise Monoethanolamin oder Triethanolamin vorgenommen werden kann.

Ihre Anwendung erfolgt in üblicher Weise durch Aufbringen einer für die Haarfärbung ausreichender Menge des Mittels auf die Haare, mit denen es eine Zeitlang, etwa 5 bis 30 Minuten, in Berührung bleibt. Anschließend wird mit Wasser, gegebenenfalls noch mit der wäßrigen Lösung einer schwachen organischen Säure, gespült und getrocknet. Als schwache organische Säure können beispielsweise Essigsäure, Zitronensäure, Weinsäure und ähnliche Verwendung finden.

Die vorstehend beschriebenen Haarfärbemittel ohne Oxidationsmittel-Zugabe können selbstverständlich auch kosmetische Polymerisate enthalten, wodurch gleichzeitig mit der Färbung eine Festigung der Haare erreicht wird. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbindungenwie Acrylsäure- bzw. Methacrylsäurepolymerisate, basische Polymerisate von Estern aus diesen beiden Säuren und Aminoalkoholen bzw. deren Salze oder Quaternisierungsprodukte, Polyacrylnitril, Polyvinyllactame sowie Copolymerisate aus derartigen Verbindungen wie Polyvinylpyrrolidon-Vinylacetat und dergleichen erwähnt.

Auch natürliche Polymere, wie Chitosan (entacetyliertes Chitin) oder Chitosanderivate, können für den genannten Zweck eingesetzt werden.

Die Polymerisate sind in diesen Mitteln in der üblichen Menge von 1 bis 5 Gew.% enthalten. Die pH-Werte der Mittel liegen im Bereich von etwa 6,0 bis 9,0.

Die Anwendung dieser Haarfärbemittel mit zusätzlicher Festigung erfolgt in bekannter und üblicher Weise durch Befeuchten des Haares mit dem Festiger, Festlegen (Einlegen, des Haares zur Frisur und anschließende Trocknung.

Selbstverständlich können die vorstehend beschriebenen Haarfärbemittel ohne Oxidationsmittel-Zugabe gegebenenfalls weitere für Haarfärbemittel übliche Zusätze wie zum Beispiel Pflegestoffe, Netzmittel, Verdicker, Weichmacher, Konservierungsstoffe und Parfümöle sowie auch andere, nachstehend für Oxidationshaarfärbemittel aufgeführte übliche Zusätze enthalten.

Zum Gegenstand der vorliegenden Erfindung gehören auch, wie eingangs erwähnt, diejenigen Haarfärbemittel, bei denen die Zugabe eines Oxidationsmittels erforderlich ist. Sie enthalten außer den Farbstoffen gemäß der angegebenen Formel I und gegebenenfalls bekannten direkt auf das Haar aufziehenden Farbstoffen noch zusätzliche bekannte Oxidationsfarbstoffe, die einer oxidativen Entwicklung bedürfen.

Bei diesen Oxidationsfarbstoffen handelt es sich hauptsächlich und aromatische p-Diamine und p-Aminophenole wie beispielsweise p-Toluylendiamin, p-Phenylendiamin, p-Aminophenol und ähnliche Verbindungen, welche zum Zwecke der Nuancierung der Färbungen mit sogenannten Modifiern, wie zum Beispiel m-Phenylendiamin, Resorcin, m-Aminophenol und anderen kombiniert werden.

Derartige zur Haarfärbung bekannte und übliche Oxidationsfarbstoffe sind unter anderem in dem Buch von E. Sagarin, "Cosmetics", Science und Technology (1957), Interscience Publishers Inc., New York, Seiten 503 ff. sowie in dem Buch von H. Janistyn, "Handbuch der Kosmetika und Riechstoffe" (1973) Seiten 338 ff., beschrieben.

Mit Mischungen aus diesen Oxidationsfarbstoffen und den Farbstoffen gemäß der angegebenen Formel I lassen sich sehr gut natürliche Blond- und Brauntöne, aber auch modische Nuancen herstellen.

Die Farbstoffe gemäß der Formel I oder II sind in diesen Färbemitteln mit Oxidationsmittel-Zugabe in einer Konzentration von etwa 0,01 bis 2,0 Gew.%, vorzugsweise 0,01 bis 1,0 Gew.%, enthalten. Der Gesamtgehalt an Farbstoffen in diesen Färbemitteln beträgt etwa 0,1 bis 5,0 Gew.%.

Oxidationshaarfärbemittel sind im allgemeinen alkalisch eingestellt, vorzugsweise auf pH-Werte von etwa 8,0 bis 11,5, wobei die Einstellung insbesondere mit Ammoniak erfolgt. Es können dazu aber auch andere organische Amine, zum Beispiel Monoethanolamin oder Triethanolamin, Verwendung finden. Als Oxidationsmittel zur Entwicklung der Haarfärbungen kommen hauptsächlich Hydrogenperoxyd und dessen Additionsverbindungen in Betracht. Die Zubereitungsform dieser Haarfärbemittel kann die gleiche wie bei Haarfärbemitteln ohne Oxidationsmittel-Zugabe sein. Vorzugsweise ist sie die einer Creme oder eines Gels.

Übliche Zusätze in Cremes, Emulsionen oder Gelen sind zum Beispiel Lösungsmittel wie Wasser, niedere aliphatische Alkohole, beispielsweise Ethanol, Propanol, Isopropanol, Glycerin oder Glykole wie Ethylenglykol und Propylenglykol, oder auch Glykolether, weiterhin Netzmittel oder Emulgatoren aus den Klassen der anionischen, kationischen, amphoteren oder nichtionogenen oberflächenaktiven Substanzen wie

Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Bentonit, Stärke, Polyacrylsäure, Cellulosederivate wie Carboxymethylcellulose, Alginate, Vaseline, Parraffinöl und Fettsäuren sowie außerdem Pflegestoffe wie Lanolinderivate, Cholesterin, Pantothensäure und Betain, weiterhin Parfümöle und Komplexbildner. Die erwähnten Bestandteile werden in den für solche Zwecke üblichen Mengen verwendet, zum Beispiel die Netzmittel und Emulgatoren in Konzentrationen von etwa 0,5 bis 30 Gew.%, während die Verdicker in einer Menge von etwa 0,1 bis 25 Gew.% in den Zubereitungen enthalten sein können.

Die Anwendung der genannten Zubereitungen, bei denen die Zugabe eines Oxidationsmittels erforderlich ist, erfolgt in bekannter Weise, indem man die Haarfärbemittel vor der Behandlung mit dem Oxidationsmittel vermischt und eine zur Färbung des Haares ausreichende Menge der Mischung, im allgemeinen etwa 50 bis 150 ml, auf das Haar aufträgt. Nach einer für die Haarfärbung ausreichenden Einwirkungszeit, welche üblicherweise etwa 10 bis 45 Minuten beträgt, wird mit Wasser, gegebenenfalls anschließend mit der wäßrigen Lösung einer schwachen organischen Säure, wie zum Beispiel Zitronensäure oder Weinsäure, gespült und getrocknet.

Hinsichtlich der färberischen Möglichkeiten bieten die erfindungsgemäßen Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt. Hierbei werden die Färbemittel je nach Zusammensetzung entweder in Verbindung mit Hydrogenperoxid oder auch ohne Oxidationsmittel angewandt.

Die 2-Nitroanilinderivate der Formeln I und II sind zum Teil bekannt. So ist zum Beispiel für folgende Verbindungen der Formel I die Herstellung in der Literatur beschrieben:

| Substituenten X | $R^1$ | $R^2$ | Literatur |
|---|---|---|---|
| $CH_3$ | H | H | L. Gattermann, Ber.Dtsch.Chem. Ges. 18, 1483 (1885) |
| $CH_3$ | H | $C_2H_4OH$ | S. Matsukawa, J. Pharm. Soc. Japan 63, 370-72 (1943) |

(Fortsetzung Tabelle)

| | | | |
|---|---|---|---|
| $CF_3$ | H | H | US-PS 2 056 899 |
| Cl | H | H | F. Beilstein et al., Liebigs Ann. Chem. 182, 99 (1876) |
| Cl | H | $C_2H_4OH$ | B.N. Feitelson et al., J. Chem. Soc. (London), 2389 (1952) |
| Br | H | $C_2H_4OH$ | |
| Br | H | $CH_2\overset{OH}{C}H-\overset{OH}{C}H_2$ | |
| Br | H | H | V. Meyer et al., Ber. Dtsch. Chem. Ges. 5, 632 (1872) |
| Br | H | $CH_3$ | J.J. Blanksma, Rec. Trav. Chim. Pays-Bas 21, 273 (1902) |
| $C_4H_9$ | H | H | C. Gelzer, Ber. Dtsch. Chem. Ges. 20, 3253 (1887) |
| $CH_2OH$ | H | H | J. Meyer et al., Ber. Dtsch. Chem. Ges. 33, 250 (1900) |

Gegenstand der vorligenden Erfindung sind weiterhin neue 2-Nitroanilinderivate der allgemeinen Formel III

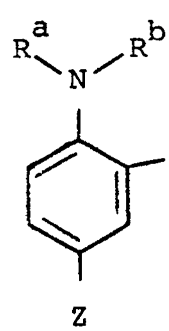

(III),

worin $R^a$ und $R^b$ unabhängig voneinander Ethyl, $C_2$-$C_4$-Monohydroxyalkyl oder $C_3$-$C_4$-Dihydroxyalkyl

bedeuten, wenn Z = $CH_3$, Cl oder Br ist, oder aber worin $R^a$ die Bedeutung H, $C_2$-$C_4$-Monohydroxyalkyl oder $C_3$-$C_4$-Dihydroxyalkyl hat und $R^b$ einen Rest 2-Hydroxyethyl oder 2,3-Dihydroxypropyl darstellt, wenn Z = $CH_2OH$, oder aber worin $R^a$ die Bedeutung H, $C_2$-$C_4$-Monohydroxyalkyl oder $C_3$-$C_4$-Dihydroxyalkyl hat und $R^b$ 2,3-Dihydroxypropyl ist, wenn Z Perfluoralkyl ist.

Beispiele für neue 2-Nitroanilinderivate der Formel III sind
4-[Bis-(2'-hydroxyethyl)-amino]-3-nitro-toluol,
4-[N-Ethyl, N-(2'-hydroxyethyl)-amino]-3-nitro-toluol,
4-(2'-Hydroxyethyl)-amino-3-nitro-benzylalkohol,
4-[(2',3'-Dihydroxypropyl)-amino]-3-nitro-1-trifluormethyl-benzol,
4-(2'Hydroxyethyl)-amino-3-nitro-1-trifluormethyl-benzol,
4-[Bis-(2'-hydroxyethyl)-amino]-3-nitro-chlorbenzol und
4-[N-Ethyl, N-(2'hydroxyethyl)-amino]-3-nitro-chlorbenzol.

Die Herstellung der neuen Verbindungen der Formel III erfolgt durch nucleophilen Austausch einer Alkoxygruppe oder eines Halogenatoms (A) in der entsprechenden, mit Z substituierten Verbindung IV gemäß der nachfolgenden Reaktionsgleichung.

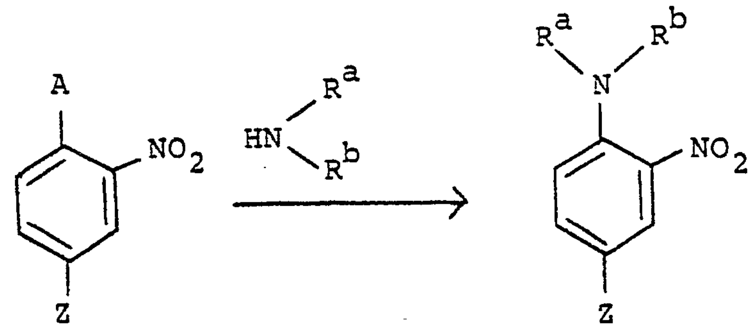

(IV) (III)

Die Herstellung der 2-Nitroanilinderivate der Formeln I bis III ist ferner möglich durch Nitrierung der gegebenenfalls geschützten p-substituierten Aniline. Zur Herstellung der 4-Chlor oder 4-Brom-Derivate des 2-Nitroanilins können ferner die entsprechenden o-Nitroaniline bromiert oder chloriert werden.

Die nachfolgenden Beispiele sollen den Gegenstand der Anmeldung weiter erläutern.

**HERSTELLUNGSBEISPIELE**

Beispiel 1 Herstellung von 4-[Bis-(2'-hydroxyethyl)-amino]-3-nitro-toluol

1,7 g (10 mmol) 4-Chlor-3-nitro-toluol werden in 15 ml Diethanolamin gelöst und 18 Stunden lang auf 135° C erhitzt. Anschließend wird auf Eis gegossen und mit Essigester extrahiert. Die vereinigten Essigesterphasen werden über Natriumsulfat getrocknet, und das Lösungsmittel wird im Vakuum verdampft. Die gesuchte Nitroverbindung bleibt als rotes Öl zurück, das noch mit geringen Mengen an Nebenprodukten verunreinigt ist. Eine Reinigung ist möglich durch Chromatographie an Kieselgel (Laufmittel Methylenchlorid mit 5% Methanol).

CH-Analyse:

| $C_{11}H_{16}N_2O_4$ | %C | %H |
|---|---|---|
| berechnet: | 54,99 | 6,71 |
| gefunden: | 54,79 | 6,55 |

NMR:
7,50 (s, breit, 2-H), 7,33 und 7,32 (2xs, 5-H, 6-H), 3,65 (t, J=5Hz, $-CH_2OH$), 3,28 (t, J=5Hz, $-N-CH_2-$), 2,38 (s, $CH_3$), 2,55-2,25 (breit, OH), 1,57 (breit, $H_2O$).
Für dieses und alle folgenden NMR-Spektren gilt:
Alle Angaben in δ[ppm]
Standard: Tetramethylsilan
Lösungsmittel: $CDCl_3$, wenn nicht anders angegeben
s = Singulett, d = Duplett, t = Triplett,
q = Quartett, m = Multiplett
UV-Spektrum: 251,6 (3,96), 447,5 (2,94)
Für dieses und alle folgenden UV-Spektren gilt:
Lösungsmittel: Wasser
Angabe in λ max [nm] (lg ε)

Beispiel 2 Herstellung von 4-[N-Ethyl, N-(2'-hydroxyethyl)-amino ]-3-nitro-toluol

1,7 g (10 mmol) 4-Chlor-3-nitro-toluol werden wie in Beispiel 1 24 Stunden lang in 5,9 ml (5,4 g, 60 mmol) 2-Ethyl-aminoethanol auf 120 °C erhitzt. Die Aufarbeitung wie in Beispiel 1 ergibt ein rotes Öl, das ebenfalls durch Chromatographie an Kieselgel gereinigt wird.
NMR:
7,47 (m, 2-H), 7,3-7,2 (m, 5-H, 6-H), 3,62 (t, J=5Hz, $-CH_2-OH$), 3,28 (t, J=5Hz, $-N-CH_2,CH_2OH$), 3,04 (q, J=7Hz, $-N-CH_2CH_3$), 2,35 (s, $CH_3$), 0,98 (t, J= 7Hz, $-N-CH_2CH_3$).
UV-Spektrum:
250,2 (3,90), 447,4 (2,85).

Beispiel 3 Herstellung von 4-(2'-Hydroxyethyl)-amino-3-nitro-benzylalkohol

1,0 g (5,3 mmol) 4-Chlor-3-nitro-benzylalkohol werden mit 5 ml Ethanolamin auf 140 °C erwärmt. Nach 3 Stunden wird der Ansatz abgekühlt und mit 15 ml Wasser versetzt. Das Produkt wird mit 3 x 40 ml Diethylether extrahiert. Die Etherphase wird einmal mit 20 ml gesättigter Kochsalzlösung gewaschen und über Magnesiumsulfat getrocknet. Nach den Abdestillieren des Dietylethers bleiben 0,84 g (4,0 mmol; 74% der Theorie) gelbes Produkt zurück. Das Produkt schmilzt bei 89-90 °C.

CHN-Analyse:

| $C_9H_{12}N_2O_4$ | %C | %H | %N |
|---|---|---|---|
| berechnet: | 50,94 | 5,70 | 13,20 |
| gefunden: | 51,17 | 5,78 | 13,11 |

Beispiel 4 Herstellung von 4-[2',3'-Dihydroxypropyl) amino]-3-nitro-1-trifluormethyl-benzol

23 g (0,10 mol) 4-Chlor-3-nitro-1-trifluormethyl-benzol werden mit 30 g 1-Amino-2,3-propandiol 3 Stunden lang auf 100 °C erwärmt. Nach dem Abkühlen wird das Produkt mit Wasser ausgefällt, abgesaugt und getrocknet. Man erhält 26,7 g (93% der Theorie) eines gelben Produktes vom Schmelzpunkt 117-118 °C.

CHN-Analyse:

| $C_{10}H_{11}F_3N_2O_4$ | %C | %H | %N |
| --- | --- | --- | --- |
| berechnet: | 42,87 | 3,96 | 10,00 |
| gefunden: | 42,87 | 3,98 | 9,99 |

Beispiel 5 Herstellung von 4-(2'-Hydroxyethyl)-amino-3-nitro-1-trifluormethyl-benzol

23 g (0,10 mol) 4-Chlor-3-nitro-1-trifluormethyl-benzol werden mit 100 ml Ethanolamin 3 Stunden lang auf 120 °C erwärmt. Nach dem Abkühlen wird das Reaktionsprodukt mit Wasser ausgefällt, abgesaugt und getrocknet. Man erhält 23 g (92% der Theorie) eines gelben Produktes vom Schmelzpunkt 72 - 74 °C.

CHN-Analyse:

| $C_9H_9F_3N_2O_3$ | %C | %H | %N |
| --- | --- | --- | --- |
| berechnet: | 43,21 | 3,63 | 11,20 |
| gefunden: | 43,14 | 3,63 | 11,29 |

Beispiel 6 Herstellung von 4-[Bis-(2'-hydroxyethyl)-amino]-3-nitro-chlorbenzol

4,2 g (22 mmol) 2,5-Dichlor-nitrobenzol werden 7 Stunden lang in 12,8 ml (14,0 g, 133 mmol) Diethanolamin auf 120 °C erhitzt. Das Reaktionsgemisch wird auf Eiswasser gegossen und mehrmals mit Essigester extrahiert. Die vereinigten Essigesterphasen werden über Natriumsulfat getrocknet und das Lösungsmittel sodann im Vakuum verdampft. Das erhaltene orangefarbene Öl kann durch Chromatographie an Kieselgel (Methylenchlorid mit 2,5 % Methanol) gereinigt werden. Die reine Verbindung kristallisiert jedoch auch bei längerem Stehen nicht. Ausbeute 2,5 g (44 % der Theorie).

| CHN-Analyse: | %C | %H | %N |
| --- | --- | --- | --- |
| $C_{10}H_{13}N_2O_4Cl$ | | | |
| berechnet: | 46,07 | 5,02 | 10,74 |
| gefunden: | 46,46 | 5,06 | 10,59 |

Beispiel 7 Herstellung von 4-[N-Ethyl, N-(2'-hydroxyethyl)-amino]-3-nitro-chlorbenzol

4,2 g (22 mmol) 2,5-Dichlor-nitrobenzol werden wie in Beispiel 6 mit 11,8 ml (10,8 g; 120 mmol) 2-Ethylaminoethanolumgesetzt. Die Aufarbeitung wie in Beispiel 6 ergibt 2,8 g (53 % der Theorie, nach Reinigung) eines viskosen roten Öls.

CHN-Analyse:

| $C_{10}H_{13}N_2O_3Cl$ | %C | %H | %N |
|---|---|---|---|
| berechnet: | 49,08 | 5,35 | 11,44 |
| gefunden: | 49,10 | 5,35 | 11,50 |

**HAARFÄRBEBEISPIELE**

Beispiel 8 Flüssiges Haarfärbemittel

| | |
|---|---|
| 0,30 g | 4-(2'-Hydroxyethyl)-amino-3-nitro-toluol |
| 2,00 g | Laurylalkoholdiglykolethersulfat-Natriumsalz (28%-ige wäßrige Lösung) |
| 2,00 g | Ammoniak, 25%-ige wäßrige Lösung |
| 95,70 g | Wasser |
| 100,00 g | |

Gebleichtes Naturhaar wird 20 Minuten lang bei Raumtemperatur mit der Lösung nach Beispiel 8 behandelt, danach mit Wasser ausgespült und getrocknet. Das Haar ist gelborange gefärbt.

Beispiel 9 Farbfestiger

| | |
|---|---|
| 0,10 g | 4-[(2',3'-Dihydroxypropyl)-amino]-3-nitro-1-trifluormethyl-benzol |
| 2,00 g | Polyvinylpyrrolidon |
| 0,10 g | Glyzerin |
| 40,00 g | Isopropanol |
| 57,80 g | Wasser |
| 100,00 g | |

Weiße menschliche Haare werden mit der Farbfestiger-Lösung eingelegt und getrocknet. Das Haar ist leuchtend zitronengelb gefärbt und gefestigt.

Beispiel 10 Oxidationshaarfärbemittel in Cremeform

| | |
|---|---|
| 0,02 g | 4-Amino-3-nitro-chlorbenzol |
| 0,20 g | p-Phenylendiamin |
| 0,15 g | Resorcin |
| 0,03 g | m-Aminophenol |
| 15,00 g | Cetylalkohol |
| 3,50 g | Laurylalkoholdiglykolethersulfat-Natriumsalz (28%-ige wäßrige Lösung) |
| 6,00 g | Ammoniak, 25%-ig |
| 75,10 g | Wasser |
| 100,00 g | |

50 g des vorstehenden Haarfärbemittels werden kurz vor der Anwendung mit 50 ml Wasserstoffperoxidlösung (6%-ig) gemischt. Das Gemisch wird anschließend auf graue menschliche Haare aufgetragen und 30 Minuten bei einer Temperatur von 40 °C einwirken gelassen. Nach dem Spülen des Haares mit Wasser und dem anschließenden Trocknen hat es einen natürlichen Blondton angenommen.

Beispiel 11 Haarfärbemittel in Cremeform

| | |
|---|---|
| 0,050 g | 4-(2'-Hydroxyethyl)-amino-3-nitro-chlorbenzol |
| 0,250 g | 4-Bis [(2'-hydroxyethyl)-amino]-1-methylamino-2-nitro-benzol |
| 0,020 g | 1-Amino-2-nitro-4-bis-(2'-hydroxyethyl)-amino-benzol |
| 0,025 g | Disperse Blue 23 (C.I. 61 545) |
| 7,500 g | Cetylalkohol |

(Fortsetzung Beispiel 11)

| | |
|---|---|
| 1,750 g | Laurylalkohol-diglykolethersulfat-Natrium-salz (28%-ige wäßrige Lösung) |
| 0,100 g | p-Hydroxybenzoesäuremethylester |
| 0,200 g | Ammoniak, 25% ig |
| 90,105 g | Wasser |
| 100,000 g | |

50 g des vorstehenden Haarfärbemittels werden auf weiße menschliche Haare aufgebracht und nach einer Einwirkungszeit von 20 Minuten mit Wasser ausgespült. Das Haar wird anschließend getrocknet. Es ist in einem natürlichen braunen Farbton gefärbt.

Beispiel 12 Flüssiges Haarfärbemittel

| | |
|---|---|
| 0,25 g | 4-(2'3'-Dihydroxypropyl)-amino-3-nitro-brombenzol |
| 0,10 g | 1-Amino-4-(2',3'-dihydroxypropyl)-amino-2-nitro-5-chlor-benzol |
| 0,20 g | 4-Bis-[(2'-hydroxyethyl)-amino]-1-methylamino-2-nitro-benzol |
| 0,50 g | Hydroxyethylcellulose |
| 5,00 g | Laurylalkoholdiglykolethersulfat-Natriumsalz (28%-ige wäßrige Lösung) |
| 10,00 g | Isopropanol |
| 10,00 g | Ammoniak, 25%-ig |
| 73,95 g | Wasser |
| 100,00 g | |

Gebleichtes Naturhaar wird 20 Minuten lang bei Raumtemperatur mit der Lösung nach Beispiel 12 behandelt. Nach Spülung mit Wasser und Trocknung ist das Haar in einem modischen dunklen Beaujolaiston eingefärbt.

Beispiel 13 Flüssiges Haarfärbemittel

| | |
|---|---|
| 0,30 g | 4-(2'-Hydroxyethyl)-amino-3-nitro-benzylalkohol |
| 0,05 g | 1,4-Bis-(2'-hydroxyethyl)-amino-2-nitro-benzol |
| 0,50 g | Hydroxyethylcellulose |
| 5,00 g | Laurylalkoholdiglykolethersulfat-Natriumsalz (28%-ige wäßrige Lösung) |
| 10,00 g | Isopropanol |
| 10,00 g | Ammoniak, 25%-ig |
| 74,15 g | Wasser |
| 100,00 g | |

Das vorstehende Färbemittel wird auf gebleichte Naturhaare 30 Minuten lang bei 40°C einwirken gelassen. Nach Spülung mit Wasser und Trocknung ist das Haar in einem modischen Blondton gefärbt.

## Ansprüche

**1.** Verwendung von 2-Nitroanilinderivaten der allgemeinen Formel I

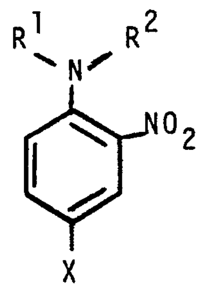

(I),

wobei $R^1$ und $R^2$ die Bedeutung $H, C_1$-$C_4$-Alkyl, $C_2$-$C_4$-Monohydroxyalkyl oder $C_3$-$C_4$-Dihydroxyalkyl haben - unter der Voraussetzung, daß $R^1$ und $R^2$ nicht gleichzeitig $C_1$-$C_4$ -Alkylreste darstellen - und X einen der Reste Alkyl, Monohydroxyalkyl, Perfluoralkyl oder Halogen bedeutet, als Farbstoff in Haarfärbemitteln.

2. Verwendung von 2-Nitroanilinderivaten der allgemeinen Formel II

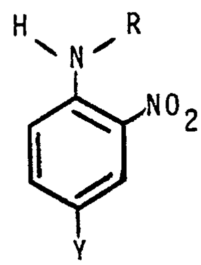

(II),

wobei R die Bedeutung 2-Hydroxyethyl oder 2,3-Dihydroxypropyl hat und Y einen der Reste $CH_3$, $CF_3$, $CH_2OH$, Cl oder Br darstellt, als Farbstoff in Haarfärbemitteln.

3. Mittel zur Färbung von Haaren mit einem Farbstoffgehalt und für Haarfärbemittel üblichen Zusätzen, dadurch gekennzeichnet, daß es 0,01 bis 2,0 Gew. % eines 2-Nitroanilinderivates der Formel I enthält.

4. Mittel nach Anspruch 3, dadurch gekennzeichnet, daß das 2-Nitroanilinderivat der Formel I ausgewählt ist aus 4-Amino-3-nitro-toloul, 4-Amino-3-nitro-benzylalkohol, 4-Amino-3-nitro-1-triflourmethyl-benzol, 4-Amino-3-nitro-chlorbenzol und 4-Amino-3-nitro-brombenzol.

5. Mittel zur Färbung von Haaren mit einem Farbstoffgehalt und für Haarfärbemittel üblichen Zusätzen, dadurch gekennzeichnet, daß es 0,0l bis 1,0 Gew. % eines 2-Nitro-anilinderivates der Formel II enthält.

6. Mittel nach Anspruch 5, dadurch gekennzeichnet, daß das 2-Nitroanilinderivat der Formel II ausgewählt ist aus 4-(2'-Hydroxyethyl)-amino-3-nitro-toluol, 4-(2'-Hydroxyethyl)-amino-3-nitro-benzylalkohol, 4-(2'-Hydroxyethyl)-amino-3-nitro-1-trifluormethyl-benzol, 4-(2',3'-Dihydroxypropyl)-amino-3-nitro-1-trifluormethyl-benzol,4-(2'-Hydroxyethyl)-amino-3-nitro-chlorbenzol, 4-(2'-Hydroxyethyl)-amino-3-nitro-brombenzol und 4-(2;3'-Dihydroxypropyl)-amino-3-nitro-brombenzol.

7. Mittel nach Anspruch 3 bis 6, zwecks Haarfestigung zusätzlich enthaltend in wäßrig - alkoholischer Lösung bekannte, direkt auf das Haar aufziehende Farbstoffe und mindestens ein kosmetisches Polymerisat.

8. Mittel nach Anspruch 7, dadurch gekennzeichnet, daß die direkt auf das Haar aufziehenden Farbstoffe ausgewählt sind aus 2-Amino-4-nitro-phenol, Pikraminsäure, 1-[(2'-Hydroxyethyl)-amino]-2-amino-4-nitro-benzol, 2-Nitro-4-[(2'-hydroxyethyl)amino]-anilin, 4-Bis-[(2'-hydroxyethyl)-amino]-1-methylamino-2-nitro-benzol, 2,5-Bis-[(2'-hydroxyethyl)-amino]-nitrobenzol, 2-(2'-Hydroxyethyl)-amino-4,6-dinitro-phenol, 1-Amino-4-(2',3'-dihydroxypropyl)-amino-2-nitro-5-chlor-benzol, Basic Violet 1 (C.I. 42 535), Acid Brown 4 (C.I. 14 805) und Disperse Violett 4 (C.I. 61 105).

9. Mittel nach den Ansprüchen 3 bis 6, zusätzlich enthaltend mindestens einen bekannten Oxidationshaarfarbstoff.

10. 2-Nitroanilinderivat der Formel III

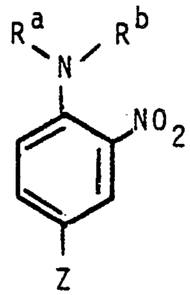

(III),

worin $R^a$ und $R^b$ unabhängig voneinander Ethyl , $C_2$-$C_4$-Monohydroxyalkyl oder $C_3$-$C_4$-Dihydroxyalkyl bedeuten, wenn Z = $CH_3$, Cl oder Br ist, oder aber worin $R^a$ die Bedeutung H, $C_2$-$C_4$-Monohydroxyalkyl oder $C_3$-$C_4$-Dihydroxyalkyl hat und $R^b$ einen Rest 2 -Hydroxyethyl oder 2 ,3 - Dihydroxypropyl darstellt, wenn Z = $CH_2OH$ ist, oder aber worin $R^a$ die Bedeutung H, $C_2$-$C_4$-Monohydroxyalkyl oder $C_3$-$C_4$-Dihydroxyalkyl hat und $R^b$ 2,3-Dihydroxypropyl ist, wenn Z Perfluoralkyl ist.

11. 4-[Bis-(2'-hydroxyethyl)-amino]-3-nitro-toluol,

12. 4-[N-Ethyl,N-(2'-hydroxyethyl)-amino]-3-nitro-toluol.

13. 4-(2'-Hydroxyethyl)-amino-3-nitro-benzylalkohol.

14. 4-[(2',3'-Dihydroxypropyl)-amino]-3-nitro-1-trifluormethyl-benzol.

15. 4-[Bis-(2'-hydroxyethyl)-amino]-3-nitro-chlorbenzol.

16. 4-[N-Ethyl,N-(2'-hydroxyethyl)-amino]-3-nitro-chlorbenzol.

## Claims

1. Use of 2-nitroaniline derivatives of the general formula I

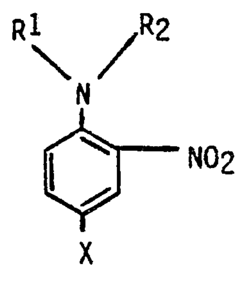

(I),

wherein $R^1$ and $R^2$ denote H, $C_1$-$C_4$-alkyl, $C_2$-$C_4$-monohydroxyalkyl or $C_3$-$C_4$-dihydroxyalkyl - provided that $R^1$ and $R^2$ do not simultaneously represent $C_1$-$C_4$-alkyl - and X denotes alkyl, perfluoroalkyl or halogen, as a dye in compositions for dyeing hair

2. Use of 2-nitroaniline derivatives of the general formula II

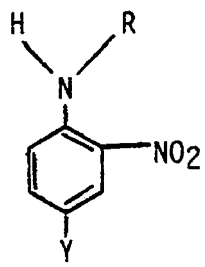

(II),

wherein R denotes 2-hydroxyethyl or 2,3-dihydroxypropyl and Y denotes $CH_3$, $CF_3$, $CH_2OH$, Cl, or Br, as a dye in compositions for dyeing hair.

3. Composition for dyeing hair with a dye content and additives customary in hair dyes, characterised in that it contains 0.01 to 2.0 % by weight of a 2-nitroaniline derivative of formula I.

4. Composition according to claim 3, characterised in that the 2-nitroaniline derivative of formula I is selected from the group consisting of 4-amino-3-nitrotoluene, 4-amino-3-nitrobenzyl alcohol, 4-amino-3-nitro-1-trifluoromethyl-benzene, 4-amino-3-nitro-chlorobenzene and 4-amino-3-nitrobromobenzene.

5. Composition for dyeing hair with a dye content and additives customary in hair dyes, characterised in that it contains 0.01 to 1.0 % by weight of a 2-nitro-aniline derivative of formula II.

6. Composition according claim 5, characterised in that the 2-nitroaniline derivative of formula II is selected from the group consisting of 4-(2'-hydroxyethyl)-amino-3-nitrotoluene, 4-(2'-hydroxyethyl)-amino-3-nitrobenzyl alcohol, 4-(2'-hydroxyethyl)-amino-3-nitro-1-trifluoromethyl-benzene, 4-(2'-hydroxyethyl)-amino-3-nitro-bromobenzene and 4-(2',3'-dihydroxypropyl)-amino-3-nitro-bromobenzene.

7. Composition according to claims 3 to 6, for the purpose of hair setting additionally containing in an aqueous-alcoholic solution known direct dyes and at least one cosmetic polymerisate.

8. Composition according to claim 7, characterised in that the direct dye is selected from the group consisting of 2-amino-4-nitrophenol, picramic acid, 1-((2'-hydroxyethyl)-amino)-2-amino-4-nitro-benzene, 2-nitro-4-((2'-hydroxyethyl)-amino)-aniline, 4-bis-((2'-hydroxyethyl)amino)-1-methylamino-2-nitro-benzene, 2,5-bis-((2'-hydroxyethyl)-amino)-nitro-benzene, 2-((2'-hydroxyethyl)-amino)-4,6-dinitrophenol, 1-amino-4-((2',3'-dihydroxypropyl)-amino)-2-nitro-5-chlorobenzene, Basic Violet 1 (C.I. 42,535), Acid Brown 4 (C.I. 14,805) and Disperse Violet 4 (C.I. 61,105).

9. Composition according to claims 3 to 6, additionally containing at least one oxidation hair dye.

10. 2-Nitroaniline derivative of formula III

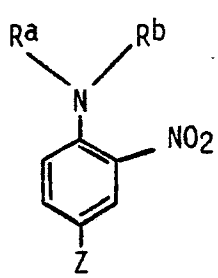

(III),

wherein $R^a$ and $R^b$, independently selected from each other, represent ethyl, $C_2$-$C_4$-monohydroxyalkyl or $C_3$-$C_4$-dihydroxyalkyl if Z is $CH_3$, Cl or Br, or wherein $R^a$ represents H, $C_2$-$C_4$-monohydroxyalkyl or $C_3$-$C_4$-dihydroxyalkyl and $R^b$ represents 2-hydroxyethyl or 2,3-dihydroxypropyl if Z is $CH_2OH$, or wherein $R^a$ represents H, $C_2$-$C_4$-monohydroxyalkyl or $C_3$-$C_4$-dihydroxyalkyl and $R^b$ represents 2,3-dihydroxypropyl if Z is perfluoroalkyl.

**11.** 4-(bis-(2'-hydroxyethyl)-amino)-3-nitro-toluene.

**12.** 4-(N-ethyl, N-(2'hyxdroxyethyl)-amino)-3-nitro-toluene.

**13.** 4-(2'-hydroxyethyl)-amino-3-nitro-benzylalcohol.

**14.** 4-((2',3'-dihydroxypropyl)-amino)-3-nitro-1-trifluoromethyl-benzene.

**15.** 4-(bis-(2'-hydroxyethyl)-amino)-3-nitro-chlorobenzene.

**16.** 4-(N-ethyl,N-(2'-hydroxyethyl)-amino)-3-nitro-chlorobenzene.

**Revendications**

**1.** Utilisation de dérivés de la 2-nitroaniline répondant à la formule générale I

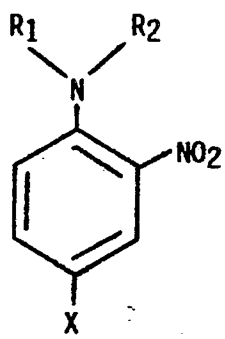

(I)

dans laquelle $R^1$ et $R^2$ représentent H, un alcoyle en $C_1$ à $C_4$, un monohydroxyalcoyle en $C_2$ à $C_4$ ou un dihydroxyalcoyle en $C_3$ à $C_4$ (avec pour condition que $R^1$ et $R^2$ ne représentent pas simultanément des restes alcoyle en $C_1$ à $C_4$) et X représente l'un des restes alcoyle, monohydroxyalcoyle, perfluoroalcoyle ou halogène, comme colorants dans des produits de coloration des cheveux.

**2.** Utilisation de dérivés de la 2-nitroaniline répondant à la formule générale II

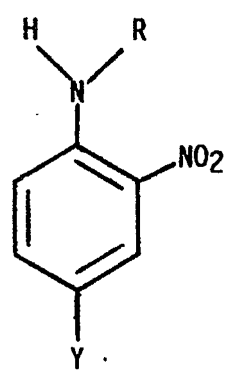

(II)

dans laquelle R représente un radical 2-hydroxyéthyle ou 2,3-dihydroxypropyle, et Y représente l'un des restes $CH_3$, $CF_3$, $CH_2OH$, Cl ou Br, comme colorant dans des produits de coloration des cheveux.

**3.** Produit de coloration des cheveux renfermant un colorant et des additifs usuels pour des produits de coloration des cheveux, caractérisé en ce qu'il renferme 0,01 à 2,0 % en poids d'un dérivé de la 2-nitroaniline répondant à la formule I.

4. Produit selon la revendication 3, caractérisé en ce que le dérivé de la 2-nitroaniline répondant à la formule I est choisi parmi le 4-amino-3-nitro-toluène, l'alcool 4-amino-3-nitro-benzylique, le 4-amino-3-nitro-1-trifluorométhyl-benzène, le 4-amino-3-nitro-chloro-benzène et le 4-amino-3-nitro-bromo-benzène.

5. Produit de coloration des cheveux renfermant un colorant et des additifs usuels pour produits de coloration des cheveux, caractérisé en ce qu'il renferme 0,01 à 1,0 % en poids d'un dérivé de la 2-nitroaniline répondant à la formule II.

6. Produit selon la revendication 5, caractérisé en ce que le dérivé de la 2-nitroaniline répondant à la formule II est choisi parmi le 4-(2'-hydroxyéthyl)-amino-3-nitro-toluène, l'alcool 4-(2'-hydroxyéthyl)-amino-3-nitro-benzylique,le 4-(2'-hydroxyéthyl)-amino-3-nitro-1-trifluorométhyl-benzène, le 4-(2',3'-dihydroxypropyl)-amino-3-nitro-1-trifluorométhyl-benzène, le 4-(2'-hydroxyéthyl)-amino-3-nitro-chlorobenzène, le 4-(2'-hydroxyéthyl)-amino-3-nitro-bromobenzène, et le 4-(2',3'-dihydroxypropyl)-amino-3-nitro-bromobenzène.

7. Produit selon les revendications 3 à 6, renfermant en plus, aux fins de fixation des cheveux, en solution hydro-alcoolique, des colorants connus prenant directement sur les cheveux et au moins un produit de polymérisation cosmétique.

8. Produit selon la revendication 7, caractérisé en ce que les colorants prenant directement sur les cheveux sont choisis parmi le 2-amino-4-nitro-phénol, l'acide picramique, le 1-((2'-hydroxyéthyl)-amino)-2-amino-4-nitrobenzène, la 2-nitro-4-((2'-hydroxyéthyl)-amino)aniline, le 4-bis-((2'-hydroxyéthyl)-amino)-1-méthylamino-2-nitrobenzène, le 2,5-bis-((2'-hydroxyéthyl)-amino)-nitrobenzène, le 2-((2'-hydroxyéthyl)-amino)-4,6-dinitrophénol, le 1-amino-4-((2',3'-dihydroxypropyl)-amino)-2-nitro-5-chlorobenzene, Basic Violet 1 (I.C. 42 535), Acid Brown 4 (I.C. 14 805) et Disperse Violet 4 (I.C. 61 105).

9. Produit selon les revendications 3 à 6, renfermant en plus, au moins un colorant d'oxydation capillaire connu.

10. Dérivé de la 2-nitroaniline répondant à la formule III

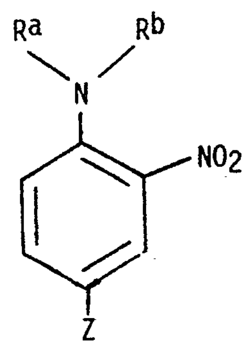

(III)

dans laquelle $R^a$ et $R^b$ représentent, indépendamment l'un de l'autre, un radical éthyle, monohydroxyalcoyle en $C_2$ à $C_4$ ou dihydroxyalcoyle en $C_3$ à $C_4$ lorsque Z = $CH_3$, Cl ou Br, ou encore dans laquelle $R^a$ représente H, un radical monohydroxyalcoyle en $C_2$ à $C_4$ ou un radical dihydroxyalcoyle en $C_3$ à $C_4$, et $R^b$ représente un reste 2-hydroxyéthyle ou 2,3-dihydroxypropyle, lorsque Z = $CH_2OH$ ou encore dans laquelle $R^a$ représente un reste H, un radical monohydroxyalcoyle en $C_2$ à $C_4$ ou un radical dihydroxyalcoyle en $C_3$ à $C_4$, et $R^b$ represente le reste 2,3-dihydroxypropyle lorsque Z = perfluoroalcoyle.

11. Le 4-(bis-(2'-hydroxyéthyl)-amino)-3-nitro-toluène.

12. Le 4-(N-éthyl, N-(2'-hydroxyéthyl)-amino)-3-nitro-toluène.

13. L'alcool 4-(2'-hydroxyéthyl)-amino-3-nitro-benzylique.

14. Le 4-((2',3'-dihydroxypropyl)-amino)-3-nitro-1-trifluorométhylbenzène.

15. Le 4-(bis-(2'-hydroxyéthyl)-amino)-3-nitro-chlorobenzène.

16. Le 4-(N-éthyl,N-(2'-hydroxyéthyl)-amino)-3-nitro-chlorobenzène.